(19) Europäisches Patentamt
European Patent Office
Office européen des brevets

(11) Veröffentlichungsnummer: **0 209 001**
**B1**

(12) # EUROPÄISCHE PATENTSCHRIFT

(45) Veröffentlichungstag der Patentschrift: **24.01.90**

(51) Int. Cl.⁴: **C07D 263/24**, C07D 413/04

(21) Anmeldenummer: **86109052.0**

(22) Anmeldetag: **03.07.86**

(54) **1-Oxa-2-oxo-3-R-3-aza-5-Z-cyclopentanderivate.**

(30) Priorität: **18.07.85 DE 3525648**

(43) Veröffentlichungstag der Anmeldung:
**21.01.87 Patentblatt 87/4**

(45) Bekanntmachung des Hinweises auf die Patenterteilung:
**24.01.90 Patentblatt 90/4**

(84) Benannte Vertragsstaaten:
**AT BE CH DE FR GB IT LI NL SE**

(56) Entgegenhaltungen:
**EP-A- 0 081 200**
**EP-A- 0 123 719**
**DE-A- 3 320 394**
**FR-A- 2 458 547**
**US-A- 3 120 510**

**Chemical Abstracts, Band 97, Nr. 19, 8. November 1982, Columbus, Ohio, USA Maruko Pharmaceutical Co., Ltd. "1-(2-Allylphenoxy)-3-isopropylaminopropan-2-ol". Seite 676, Spalte 1, Zusammenfassung-Nr. 162 573u**
**Chemical Abstracts, Band 98, Nr. 7, 14. Februar 1983, Columbus, Ohio, USA Dostert, Philippe; Strolin Benedetti; Margherita; Jalfre, Maurice "Structural modifications in oxazolidinone series leading to type A or B selective monoamine oxidase inhibitors". Seite 21, Spalte 2, Zusammenfassung-Nr. 46 460b 000**

(73) Patentinhaber: **Bokel, Heinz-Hermann, Dr., Dürerstrasse 30, D-6100 Darmstadt(DE)**

(72) Erfinder: **Bokel, Heinz-Hermann, Dr., Dürerstrasse 30, D-6100 Darmstadt(DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 1-Oxa-2-oxo-3-R-3-aza-5-Z-cyclopentanderivate (I)
worin
R Alkyl oder Hydroxyalkyl mit jeweils 1–6 C-Atomen, Cycloalkyl mit 3–8 C-Atomen, unsubstituiertes Aryl oder Aralkyl oder mit Arylrest ein- bis dreifach durch Alkyl, Alkoxy, OH und/oder Cl oder einfach durch Methylendioxy substituiertes Aryl oder Aralkyl mit jeweils insgesamt 6–15 C-Atomen,
Z $-(CHOH)_n-H$ und
n 2, 3, 4 oder 5
bedeuten.

Ähnliche Verbindungen sind in der US-PS 3 120, 510, der EP-A 81 200, der DE-A 3 320 394, der EP-A 123 719 und in Chem. Abstr. 98, 469 606 (1983) beschrieben.

Die Verbindungen I können als Zwischenprodukte zur Herstellung von Arzneimittelwirkstoffen wie Toloxaton oder bestimmten Betarezeptorenblockern verwendet werden.

Die Verbindungen I können hergestellt werden, indem man eine Verbindung der Formel $R-NH-CH_2-CHOH-Z$ (II) mit einem reaktionsfähigen Derivat der Kohlensäure umsetzt.

Verbindungen der Formel II sind teilweise bekannt und z.B. erhältlich durch Umsetzung von Aminen der Formel $R-NH_2$ mit Aldehyden der Formel Z–CHOH–CHO sowie anschließende oder gleichzeitige Reduktion der gebildeten Schiffschen Basen bzw. Halbaminale der Formeln R–N=CH–CHOH–Z bzw. $R-NH-(CHOH)_2-Z$ bzw.

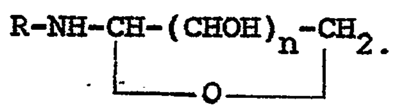

Als Amine der Formel $R-NH_2$ eignen sich z.B. Methyl-, Ethyl-, Propyl-, Isopropyl-, Butyl-, Isobutyl-, sek.-Butyl-, tert.-Butyl-, Pentyl-, 1-Ethylpropyl-, 1-Methylbutyl-, Isopentyl-, Neopentyl-, tert.-Pentyl-, Hexyl-, Isohexyl-, 1,1-Dimethylbutyl-, 1-Methylpentyl-, 2-Hydroxyethyl-, 2- oder 3-Hydroxypropyl-, 2-Hydroxy-1-methylethyl-, 2-, 3- oder 4-Hydroxybutyl-, 4-Hydroxypentyl-, 6-Hydroxyhexyl-, Cyclopropyl-, Cyclobutyl-, Cyclopentyl-, Cyclohexyl-, 1-, 2- oder 3-Methylcyclopentyl-, 1-, 2-, 3- oder 4-Methylcyclohexyl-, Cycloheptyl-, Cyclooctyl-, 2-Phenylethyl-, 1-Methyl-2-phenylethyl-, 1,1-Dimethyl-2-phenylethyl-, 2- oder 3-Phenylpropyl-, 1-Methyl-3-phenylpropyl-, 2-, 3- oder 4-Phenylbutyl-, 2-(1- oder 2-Naphthyl)-ethyl-, 2-, 3- oder 1-p-Methoxyphenylethyl-, 2-(3,4-Dimethoxyphenyl)-ethyl-, 2-(3,4-Methylendioxyphenyl)-ethyl-amin, Anilin, o-, m- oder p-Toluidin, o-, m- oder p-Anisidin, o-, m- oder p-Aminophenol, o-, m- oder p-Chloranilin, 3,4-Dimethoxyanilin, 3,4-Methylendioxyanilin.

Als Aldehyde der Formel Z–CHOH–CHO eignen sich z.B. 2,3,4-Trihydroxybutanale wie die DL-, D- oder L-Formen von Erythrose oder Threose, 2,3,4,5-Tetrahydroxypentanale wie die DL-, D- oder L-Formen von Ribose, Arabinose, Xylose oder Lyxose, 2,3,4,5,6-Pentahydroxyhexalane wie die DL-, D- oder L-Formen von Allose, Altrose, Glucose, Mannose, Gulose, Idose, Galactose oder Talose. Typische Verbindungen der Formel II sind z.B. N–R-2,3,4-trihydroxybutylamine wie N-Methyl-2,3,4-trihydroxybutylamine, N–R-2,3,4,5-tetrahydroxypentylamine wie N-Phenyl-2,3,4,5-tetrahydroxypentylamine, N–R-2,3,4,5,6-pentahydroxyhexylamine wie N-Phenyl-2,3,4,5,6-pentahydoxyhexylamine, N-m-Tolyl-2,3,4,5,6-pentahydroxyhexylamine, N-Methyl-, N-Ethyl-, N-Isopropoyl- oder N-tert.-Butyl-2,3,4,5,6-pentahydroxyhexylamine, z.B. die von D-Glucose abgeleiteten N-R-glucamine (N-R-2S,3R,4R,5R,6-pentahydroxyhexylamine).

Geeignete Kohlensäurederivate sind z.B. Phosgen, Dialkylcarbonate wie Dimethyl- oder Diethylcarbonat, Harnstoff oder Carbonyldiimidazol.

Die Reaktion von II mit dem Kohlensäurederivat kann in Abwesenheit oder Anwesenheit eines inerten Lösungsmittels wie Dimethylformamid (DMF), Methanol, Ethanol, bei Temperaturen zwischen etwa 0 und etwa 200° durchgeführt werden. So arbeitet man mit Carbonyldiimidazol zweckmäßig bei etwa 0 - 30° in DMF, mit den übrigen genannten Kohlensäurederivaten bei etwa 80-150° ohne Lösungsmittel, wobei jedoch der Zusatz einer Base wie NaOH, KOH, Triethylamin oder Pyridin vorteilhaft sein kann.

In einigen Fällen können auch weitere OH-Gruppen in II mit dem Kohlensäurederivat reagieren, besonders, wenn dieses im Überschuß eingesetzt wird. Die so entstandenen Carbonate können aber leicht alkalisch verseift werden, wobei die gewünschten Verbindungen I entstehen.

Oxydative Spaltung der Verbindungen (I), z.B. mit $HJO_4$ oder deren Salzen, $KMnO_4$, Bleitetraacetat, und anschließende Reduktion der intermediär gebildeten 1-Oxa-2-oxo-3-R-3-aza-5-formylcyclopentane führt zu den entsprechenden 1-Oxa-2-oxo-3-R-3-aza-5-hydroxymethylcyclopentanen (III), z.B. zu Toloxaton (R = m-Tolyl). Verwendet man Verbindungen (I), worin das C(5)-Atom die S-Konfiguration besitzt, so erhält man die entsprechenden 1-Oxa-2-oxo-3-R-3-aza-5S-hydroxymethyl cyclopentane. Die Umwandlung der 3-Isopropylverbindung in S-Propranolol ist bekannt. Aus den Verbindungen III lassen sich durch Umsetzungen mit $SOCl_2$, $PBr_3$, durch Veresterung oder Veretherung die entsprechenden 5-Chlormethyl-, 5-Brommethyl-, 5-Acyloxymethyl-(z.B. auch 5-Methansulfonyloxymethyl-, 5-p-Toluolsulfonyloxymethyl-), 5-Alkoxymethyl- oder 5-Aryloxymethylderivate herstellen.
Alle Temperaturen sind in °C angegeben.

Beispiel 1

Eine Lösung von 2,71 g N-m-Tolyl-2S,3R,4R,5R,6-pentahydroxyhexylamin [IIa; F. 111-114° erhältlich aus D-Glucose und m-Toluidin/$H_2$/Pd-C in Methanol/Wasser] in 30 ml DMF wird mit 1,62 g Carbonyldiimidazol versetzt und 3 Stunden bei 20° gerührt. Nach Konzentrieren wird Imidazol bei 130°/0,2 bar abdestilliert. Man erhält 1-Oxa-2-oxo-3-m-tolyl-3-aza-5S-(1R,2R,3R,4-tetrahydroxybutyl)-cyclopentan (Ia) das durch Chromatographie an

Kieselgel gereinigt wird. Rf 0,45 (Dichlormethan/Methanol/Essigsäure 90:10:5).

Beispiel 2

Ein Gemisch von 2,71 g IIa, 10 ml Diethylcarbonat und 0,5 ml Triethylamin wird 23 Stunden bei 110-120° gerührt. Man dampft ein, chromatographiert an Kieselgel und erhält Ia.

Beispiel 3

Ein Gemisch von 2,71 g IIa, 0,72 g Harnstoff und 0,1 g KOH wird 2 Stunden auf 140 -150° erhitzt. Nach dem Abkühlen chromatographiert man das erhaltene Ia an Kieselgel.

Beispiel 4

Ein Gemisch von 2,71 g IIa und 4 g Ethylencarbonat wird 3 Stunden auf 100° erhitzt. Man dampft ein, chromatographiert an Kieselgel und erhält Ia.

Beispiele 5 bis 20

Analog Beispiel 1, 2, 3 oder 4 erhält man aus N-Methylglucamin, N-Ethylglucamin, N-Propylglucamin, N-Isopropylglucamin, N-Butylglucamin, N-Isobutylglucamin, N-sek.-Butylglucamin, N-tert.-Butylglucamin, N-(2-Hydroxyethyl)-glucamin, N-Cyclohexylglucamin, N-Phenylglucamin, N-p-Methoxyphenylglucamin, N-(2-Phenylethyl)-glucamin, N-(1,1-Dimethyl-2-phenylethyl)-glucamin, N-[2-(3,4-Dimethoxyphenyl)-ethyl]-glucamin bzw. N-[2-(3,4-Methylendioxyphenyl)-ethyl]-glucamin die folgenden 1-Oxa-2-oxo-3-aza-5S-(1R,2R,3R,4-tetrahydroxybutyl)-cyclopentane:

5. 3-Methyl-, F. 155°.
6. 3-Ethyl-.
7. 3-Propyl-.
8. 3-Isopropyl-.
9. 3-Butyl-.
10. 3-Isobutyl-.
11. 3-sek.-Butyl-.
12. 3-tert.-Butyl-, F. 158°.
13. 3-(2-Hydroxyethyl)-.
14. 3-Cyclohexyl-.
15. 3-Phenyl-.
16. 3-p-Methoxyphenyl-.
17. 3-(2-Phenylethyl)-.
18. 3-(1,1-Dimethyl-2-phenylethyl)-.
19. 3-[2-(3,4-Dimethoxyphenyl)-ethyl]-.
20. 3-[2-(3,4-Methylendioxyphenyl)-ethyl]-.

Beispiel 21

Analog Beispiel 2 erhält man aus N-Isopropyl-2R,3R,4R, 5R,6-pentahydroxyhexylamin (F. 122°; erhältlich durch 3stündiges Behandeln einer Lösung von D-Mannose und Isopropylamin in Methanol/Wasser mit $H_2$ an 5%ig. Pd-C bei 50° und 3 bar) das 1-Oxa-2-oxo-3-isopropyl-3-aza-5R-(1R,2R,3R,-4-tetrahydroxybutyl)-cyclopentan.

Beispiel 22

Eine Lösung von 2,37 g N-tert.-Butyl-2S,3R,4R,5R,6-pentahydroxyhexylamin (F. 112°; erhältlich aus D-Glucose und tert.-Butylamin/$H_2$/Pd-C) in 10 ml Diethylcarbonat wird mit 100 mg KOH/Pulver versetzt und 4 Stunden auf120° erhitzt. Man engt ein, nimmt in Ethanol auf und filtriert.Nach Kühlung werden die ausgefallenden Kristalle abgesaugt; F.212°. Zur Verseifung der gebildeten Carbonat-Estergruppen erwärmt man das Zwischenprodukt kurz mit einem Gemisch von 3 ml Methanol, 3 ml Wasser und 0,5 g KOH. Nach Neutralisation mit Salzsäure konzentriert man die Lösung, extrahiert den Rückstand mit Dichlormethan, dampft ein und erhält 1-Oxa-2-oxo-3-tert.-butyl-3-aza-5S-(1R,2R,3R-4-tetrahydroxybutyl)-cyclopentan, F. 158°.

Verwendungsbeispiel 1

Eine Suspension von 2,97 g Ia in 180 ml Wasser wird bei 20°mit 7,2 g $NaJO_4$ versetzt und 30 Minuten gerührt. Anschließend stellt man auf pH 8 ein und versetzt bei 20° portionsweise mit 0,2 g $NaBH_4$. Nach weiterem 1,5stündigem Rühren wird mit Dichlormethan extrahiert, der Extrakt mit $Na_2SO_4$ getrocknet und eingedampft, der Rückstand chromatographisch gereinigt. Man erhält 1-Oxa-2-oxo-3-m-tolyl-3-aza-5S-hydroxymethyl-cyclopentan.

Verwendungsbeispiele 2 bis 17

Analog Verwendungsbeispiel 1 erhält man durch oxydative Spaltung und nachfolgende Reduktion der in Beispiel 5 bis 20 genannten Verbindungen die folgenden 1-Oxa-2-oxo-3-aza-5S-hydroxymethyl-cyclopentane:

2. 3-Methyl-.
3. 3-Ethyl-.
4. 3-Propyl-.
5. 3-Isopropyl-, F. 55-58°.
6. 3-Butyl-.
7. 3-Isobutyl-.
8. 3-sek.-Butyl-.
9. 3-tert.-Butyl-.
10. 3-(2-Hydroxyethyl)-.
11. 3-Cyclohexyl-.
12. 3-Phenyl-.
13. 3-p-Methoxyphenyl-.
14. 3-(2-Phenylethyl)-.
15. 3-(1,1-Dimethyl-2-phenylethyl)-.
16. 3-[2-(3,4-Dimethoxyphenyl)-ethyl]-.
17. 3-[2-(3,4-Methylendioxyphenyl)-ethyl]-.

**Patentansprüche**

1. 1-Oxa-2-oxo-3-R-3-aza-5-Z-cyclopentanderivate (I)
worin
R Alkyl oder Hydroxyalkyl mit jeweils 1–6 C-Atomen, Cycloalkyl mit 3–8 C-Atomen, unsubstituiertes Aryl oder Aralkyl oder im Arylrest ein- bis dreifach

durch Alkyl, Alkoxy, OH und/oder Cl oder einfach durch Methylendioxy substituiertes Aryl oder Aralkyl mit jeweils insgesamt 6–15 C-Atomen,
Z $-(CHOH)_n-H$ und
n 2, 3, 4 oder 5
bedeuten.

2. Verbindungen nach Anspruch 1:
a) 1-Oxa-2-oxo-3-methyl-3-aza-5-(1,2,3,4-tetrahydroxybutyl)-cyclopentan;
b) 1-Oxa-2-oxo-2-isopropyl-3-aza-5-(1,2,3,4-tetrahydroxybutyl)-cyclopentan;
c) 1-Oxa-2-oxo-3-tert.-butyl-3-aza-5-(1,2,3,4-tetrahydroxybutyl)-cyclopentan;
d) 1-Oxa-2-oxo-3-m-tolyl-3-aza-(1,2,3,4-tetrahydroxybutyl)-cyclopentan.

3. Verfahren zur Herstellung von Verbindungen (I) nach Anspruch 1, dadurch gekennzeichnet, daß man eine Verbindung der Formel II

$$RNH-CH_2-CHOH-Z \quad II$$

in Abwesenheit oder Anwesenheit eines inerten Lösungsmittels bei Temperaturen zwischen etwa 0 und etwa 200° mit einem reaktionsfähigen Derivat der Kohlensäure umsetzt.

4. Verwendung von Verbindungen (I) nach Anspruch 1 als Zwischenprodukte zur Herstellung von 1-Oxa-2-oxo-3R-3-aza-5-hydroxymethyl-cyclopentanen durch oxydative Spaltung und anschließende Reduktion der intermediär gebildeten 1-Oxa-2-oxo-3R-3-aza-5-formyl-cyclopentane.

## Claims

1. 1-Oxa-2-oxo-3-R-3-aza-5-Z-cyclopentane derivatives (I)
wherein
R is alkyl or hydroxyalkyl having in each case 1–6 C atoms, cycloalkyl having 3–8 C atoms, unsubstituted aryl or aralkyl or aryl or aralkyl each of which has a total of 6–15 C atoms and, in the aryl radical, is monosubstituted to trisubstituted by alkyl, alkoxy, OH and/or Cl or monosubstituted by methylenedioxy,
Z is $-(CHOH)_n-H$ and
n is 2, 3, 4 or 5.

2. Compounds according to Claim 1:
a) 1-Oxa-2-oxo-3-methyl-3-aza-5-(1,2,3,4-tetrahydroxybutyl)-cyclopentane;
b) 1-Oxa-2-oxo-3-isopropyl-3-aza-5-(1,2,3,4-tetrahydroxybutyl)-cyclopentane;
c) 1-Oxa-2-oxo-3-tert.-butyl-3-aza-5-(1,2,3,4-tetrahydroxybutyl)-cyclopentane;
d) 1-Oxa-2-oxo-3-m-tolyl-3-aza-(1,2,3,4-tetrahydroxybutyl)-cyclopentane.

3. Process for the preparation of compounds (I) according to Claim 1, characterized in that a compound of the formula II

$$RNH-CH_2-CHOH-Z \quad II$$

is reacted with a reactive derivative of carbonic acid in absence or in presence of an inert solvent at temperatures between about 0 and about 200°.

4. The use of compounds (I) according to Claim 1 as intermediate products for the preparation of 1-oxa-2-oxo-3-R-3-aza-5-hydroxymethyl-cyclopentanes or oxydative cleavage and subsequent reduction of the 1-oxa-2-oxo-3-R-3-aza-5-formyl-cyclopentanes formed as intermediates.

## Revendications

1. 1-oxa-2-oxo-3-R-3-aza-5-Z-cyclopentanes I dans lesquels
R représente un groupe alkyle ou hydroxyalkyle contenant chacun à 1 à 6 atomes de carbone, un groupe cycloalkyle en C 3–C 8, aryle ou aralkyle non substitué ou aryle ou aralkyle mono- à tri-substitué dans la partie aryle par des groupes alkyle, alcoxy, OH et/ou Cl ou monosubstitué par un groupe méthylène-dioxy et contenant chacun 6 à 15 atomes de carbone au total,
Z représente $-(CHOH)_n-H$, et
n est égal à 2, 3, 4 ou 5.

2. Composés selon la revendication 1:
a) le 1-oxa-2-oxo-3-méthyl-3-aza-5-(1,2,3,4-tétrahydroxybutyl)-cyclopentane,
b) le 1-oxa-2-oxo-3-isopropyl-3-aza-5-(1,2,3,4-tétrahydroxybutyl)-cyclopentane,
c) le 1-oxa-2-oxo-3-tert-butyl-3-aza-5-(1,2,3,4-tétrahydroxybutyl)-cyclopentane,
d) le 1-oxa-2-oxo-3-m-tolyl-3-aza-(1,2,3,4-tétrahydroxybutyl)-cyclopentane.

3. Procédé de préparation des composés I selon la revendication 1, caractérisé en ce que l'on fait réagir un composé de formule II

$$RNH-CH_2-CHOH-Z \quad II$$

en l'absence ou en présence d'un solvant inerte, à des températures allant de 0 à 200° environ, avec un dérivé réactif de l'acide carbonique.

4. Utilisation des composés I selon la revendication 1 en tant que produits intermédiaires de la préparation de 1-oxa-2-oxo-3R-3-aza-5-hydroxyméthyl-cyclopentanes par scission par oxydation suivie d'une réduction des 1-oxa-2-oxo-3R-3-aza-5-formyl-cyclopentanes formés en produits intermédiaires.